# EUROPEAN PATENT APPLICATION

(11) **EP 3 572 523 A1**
(43) Date of publication of application: **27.11.2019**
(21) Application number: 18174336.0
(22) Date of filing: 25.05.2018
(51) Int. Cl.: C12P 19/34, C12N 9/18, C12N 15/10, C12N 15/63

(54) **NOVEL CARBOXYESTERHYDROLASES**

(71) Applicant: Bayer Aktiengesellschaft, 51373 Leverkusen (DE); Heinrich-Heine-Universität Düsseldorf, 40225 Düsseldorf (DE); Agencia Estalal Consejo Superior de Investigaciones Cientificas, 28006 Madrid (ES)
(72) Inventor: The designation of the inventor has not yet been filed
(74) Representative: BIP Patents

(57) **Abstract**

What is disclosed herein relates to a polypeptide selected from the group consisting of SEQ ID NO 1 to SEQ ID NO 8 or a sequence with at least 80% sequence identity to any one of said sequences, wherein said polypeptide has activity towards 3,5-dimethylphenyl 2-chlorobenzoate.

## Description

Carboxyesterhydrolases (EC 3.1.1) and especially lipases (EC 3.1.1.3) are esterases that catalyze the hydrolysis of ester bonds typically in lipids (Casas-Godoy et al. 2012; Ferrer et al. 2015; Jaeger et al. 1994).

Moreover, carboxyesterhydrolases can be employed in biocatalysis reactions where they facilitate the separation of enantiomers or the removal of protective groups under mild reaction conditions.

Even though numerous carboxyesterhydrolases are already known, there remains a need for novel carboxyesterhydrolases with altered properties that offer improved performance in various industrial applications.

Thus, it was the object of the present invention to provide novel carboxyesterhydrolases.

This object is met by the provision of a polypeptide selected from the group consisting of SEQ ID NO 1 to SEQ ID NO 8 or a sequence with at least 80% sequence identity to any one of said sequences, wherein said polypeptide has activity towards 3,5-dimethylphenyl 2-chlorobenzoate.

To the knowledge of the inventors of the current invention a cleavage of this substrate by carboxyesterhydrolases has not been described so far.

Therefore, the novel carboxyesterhydrolases offer the possibility to catalyze sterically demanding substrates.

The term "sterically demanding" refers to substrates that due to their bulky substituents influence and/or limit the interaction of the carboxyesterhydrolase with the substrate.

The term "comprising" is to be interpreted as specifying the presence of the stated parts, steps or components, but does not exclude the presence of one or more additional parts, steps or components.

It is understood that when referring to a word in the singular, the plural is also included herein. Thus, reference to an element by the indefinite article "a" or "an" does not exclude the possibility that more than one of the element is present, unless the context clearly requires that there be one and only one of the elements. The indefinite article "a" or "an" thus usually means "at least one".

As used herein the term "polypeptide" refers to any peptide, polypeptide, oligopeptide or protein. A polypeptide consists of consecutive amino acids, which are linked by peptide bonds. The polymer may be linear or branched, it may comprise modified amino acids, and it may be interrupted by non-amino acids. The polypeptide may be human, non- human, and an artificial or chemical mimetic of a corresponding naturally occurring amino acid, as well as to naturally occurring amino acid polymers and non- naturally occurring amino acid polymer. The term also encompasses an amino acid polymer that has been modified by either natural processes or by chemical modifications; for example, by disulfide bond formation, glycosylation, lipidation, acetylation, acylation, phosphorylation, or any other manipulation, such as conjugation with a labeling component, such as but not limited to, fluorescent markers, particles, biotin, beads, proteins, radioactive labels, chemiluminescent tags, bioluminescent labels, and the like.

As used herein the term "sequence identity" of two related nucleotide or amino acid sequences, expressed as a percentage, refers to the number of positions in the two optimally aligned sequences which have identical residues (x 100) divided by the number of positions compared. A gap, i.e. a position in an alignment where a residue is present in one sequence but not in the other, is regarded as a position with non-identical residues. The "optimal alignment" of two sequences is found by aligning the two sequences over the entire length. In other words if two identical sequences are aligned the sequence identity value would be 100%. Aligned sequences of nucleotide or amino acid residues are typically represented as rows within a matrix. Gaps are inserted between the residues so that identical or similar characters are aligned in successive columns. In order to determine the sequence identity the Needleman and Wunsch global alignment algorithm (Needleman and Wunsch, 1970, J Mol Biol 48(3):443-53) of The European Molecular Biology Open Software Suite (EMBOSS, Rice et al., 2000, Trends in Genetics 16(6): 276- 277; see e.g. http://www.ebi.ac.uk/emboss/align/index.html) using default settings (gap opening penalty = 10 (for nucleotides) / 10 (for proteins) and gap extension penalty = 0.5 (for nucleotides) / 0.5 (for proteins)) can be employed. For nucleotides the default scoring matrix used is EDNAFULL and for proteins the default scoring matrix is EBLOSUM62.

The term "nucleic acid molecule" is intended to indicate any single- or double stranded nucleic acid molecule comprising DNA (cDNA and/or genomic DNA), RNA (preferably mRNA), PNA, LNA and/or Morpholino.

As used herein the term "carboxyesterhydrolase" refers to an enzyme in class EC 3.1.1 as defined by Enzyme Nomenclature. For purposes of the present invention, carboxyesterhydrolaseactivity is determined according to the procedure described in Example 3 i.e. via determining hydrolytic activity towards tributyrin (Kok et al. 1993).

Thus a polypeptide with carboxyesterhydrolase activity is a polypeptide comprising a carboxyesterhydrolase. In preferred embodiments the polypeptide with carboxyesterhydrolase activity consists of said carboxyesterhydrolase.

In preferred embodiments of the polypeptide with carboxyesterhydrolase activity comprising an amino acid sequence selected from the group consisting of SEQ ID NO 1 to SEQ ID NO 8 said polypeptide has at least 80% sequence identity, more preferably at least 85% sequence identity, more preferably at least 90% sequence identity, more preferably at least 95% sequence identity, more preferably at least 96%, 97%, 98%, 99%, and most preferably 100% sequence identity to the selected sequence of the group consisting of SEQ ID NO 1 to SEQ ID NO 8.

The polypeptide with carboxyesterhydrolase activity comprising an amino acid sequence selected from the group consisting of SEQ ID NO 1 to SEQ ID NO 8 or a sequence with at least 80% sequence identity to any one of said sequences can also be characterized via its nucleic acid sequence i.e. via a polynucleotide.

Thus, the polypeptide with carboxyesterhydrolase activity comprising an amino acid sequence selected from the group consisting of SEQ ID NO 1 to SEQ ID NO 8 can be characterized in that
a) the polypeptide of SEQ ID NO 1 is encoded by the nucleic acid sequence of SEQ ID NO 9 or a nucleic acid sequence with at least 80% sequence identity to SEQ ID NO 9 and
b) the polypeptide of SEQ ID NO 2 is encoded by the nucleic acid sequence of SEQ ID NO 10 or a nucleic acid sequence with at least 80% sequence identity to SEQ ID NO 10 and
c) the polypeptide of SEQ ID NO 3 is encoded by nucleic acid sequence of SEQ ID NO 11 or a nucleic acid sequence with at least 80% sequence identity to SEQ ID NO 11 and
d) the polypeptide of SEQ ID NO 4 is encoded by the nucleic acid sequence of SEQ ID NO 12 or a nucleic acid sequence with at least 80% sequence identity to SEQ ID NO 12 and
e) the polypeptide of SEQ ID NO 5 is encoded by the nucleic acid sequence of SEQ ID NO 13 or a nucleic acid sequence with at least 80% sequence identity to SEQ ID NO 13 and
f) the polypeptide of SEQ ID NO 6 is encoded by the nucleic acid sequence of SEQ ID NO 14 or a nucleic acid sequence with at least 80% sequence identity to SEQ ID NO 14 and
g) the polypeptide of SEQ ID NO 7 is encoded by the nucleic acid sequence of SEQ ID NO 15 or a nucleic acid sequence with at least 80% sequence identity to SEQ ID NO 15 and
h) the polypeptide of SEQ ID NO 8 is encoded by the nucleic acid sequence of SEQ ID NO 16 or a nucleic acid sequence with at least 80% sequence identity to SEQ ID NO 16.

Moreover, the polypeptide with carboxyesterhydrolase activity comprising an amino acid sequence selected from the group consisting of SEQ ID NO 1 to SEQ ID NO 8 or a sequence with at least 80% sequence identity to any one of said sequences can also be characterized via the complements of the nucleic acid sequence of SEQ ID NO 9 to SEQ ID NO 16 respectively.

In addition the nucleic acid sequence of SEQ ID NO 9 to SEQ ID NO 16 can be codon optimized.

Thus, in case of the polypeptide with carboxyesterhydrolase activity comprising an amino acid sequence as set forth in SEQ ID NO. 1 the provided polypeptide is selected from the group
a) a polypeptide comprising an amino acid sequence as set forth in SEQ ID NO. 1 or a sequence with at least 80% sequence identity to said sequence,
b) an isolated codon optimized nucleic acid fragment comprising a nucleotide sequence of at least 80% sequence identity to SEQ ID NO. 9
c) an isolated nucleic acid fragment comprising a sequence complementary to SEQ ID NO. 9,
d) an isolated nucleic acid fragment comprising a sequence which specifically hybridizes to said isolated nucleic acid fragment of b) or said complementary of c).

In case of the polypeptide with carboxyesterhydrolase activity comprising an amino acid sequence as set forth in SEQ ID NO. 2 the provided polypeptide is selected from the group
a) a polypeptide comprising an amino acid sequence as set forth in SEQ ID NO. 2 or a sequence with at least 80% sequence identity to said sequence,
b) an isolated codon optimized nucleic acid fragment comprising a nucleotide sequence of at least 80% sequence identity to SEQ ID NO. 10
c) an isolated nucleic acid fragment comprising a sequence complementary to SEQ ID NO. 10,
d) an isolated nucleic acid fragment comprising a sequence which specifically hybridizes to said isolated nucleic acid fragment of b) or said complementary of c).

In case of the polypeptide with carboxyesterhydrolase activity comprising an amino acid sequence as set forth in SEQ ID NO. 3 the provided polypeptide is selected from the group
a) a polypeptide comprising an amino acid sequence as set forth in SEQ ID NO. 3 or a sequence with at least 80% sequence identity to said sequence,
b) an isolated codon optimized nucleic acid fragment comprising a nucleotide sequence of at least 80% sequence identity to SEQ ID NO. 11
c) an isolated nucleic acid fragment comprising a sequence complementary to SEQ ID NO. 11,
d) an isolated nucleic acid fragment comprising a sequence which specifically hybridizes to said isolated nucleic acid fragment of b) or said complementary of c).

In case of the polypeptide with carboxyesterhydrolase activity comprising an amino acid sequence as set forth in SEQ ID NO. 4 the provided polypeptide is selected from the group
a) a polypeptide comprising an amino acid sequence as set forth in SEQ ID NO. 4 or a sequence with at least 80% sequence identity to said sequence,
b) an isolated codon optimized nucleic acid fragment comprising a nucleotide sequence of at least 80% sequence identity to SEQ ID NO. 12
c) an isolated nucleic acid fragment comprising a sequence complementary to SEQ ID NO. 12,
d) an isolated nucleic acid fragment comprising a sequence which specifically hybridizes to said isolated nucleic acid fragment of b) or said complementary of c).

In case of the polypeptide with carboxyesterhydrolase activity comprising an amino acid sequence as set forth in SEQ ID NO. 5 the provided polypeptide is selected from the group
a) a polypeptide comprising an amino acid sequence as set forth in SEQ ID NO. 5 or a sequence with at least 80% sequence identity to said sequence,
b) an isolated codon optimized nucleic acid fragment comprising a nucleotide sequence of at least 80% sequence identity to SEQ ID NO. 13
c) an isolated nucleic acid fragment comprising a sequence complementary to SEQ ID NO. 13,
d) an isolated nucleic acid fragment comprising a sequence which specifically hybridizes to said isolated nucleic acid fragment of b) or said complementary of c).

In case of the polypeptide with carboxyesterhydrolase activity comprising an amino acid sequence as set forth in SEQ ID NO. 6 the provided polypeptide is selected from the group
a) a polypeptide comprising an amino acid sequence as set forth in SEQ ID NO. 6 or a sequence with at least 80% sequence identity to said sequence,
b) an isolated codon optimized nucleic acid fragment comprising a nucleotide sequence of at least 80% sequence identity to SEQ ID NO. 14
c) an isolated nucleic acid fragment comprising a sequence complementary to SEQ ID NO. 14,
d) an isolated nucleic acid fragment comprising a sequence which specifically hybridizes to said isolated nucleic acid fragment of b) or said complementary of c).

In case of the polypeptide with carboxyesterhydrolase activity comprising an amino acid sequence as set forth in SEQ ID NO. 7 the provided polypeptide is selected from the group
a) a polypeptide comprising an amino acid sequence as set forth in SEQ ID NO. 7 or a sequence with at least 80% sequence identity to said sequence,
b) an isolated codon optimized nucleic acid fragment comprising a nucleotide sequence of at least 80% sequence identity to SEQ ID NO. 15
c) an isolated nucleic acid fragment comprising a sequence complementary to SEQ ID NO. 15,
d) an isolated nucleic acid fragment comprising a sequence which specifically hybridizes to said isolated nucleic acid fragment of b) or said complementary of c).

In case of the polypeptide with carboxyesterhydrolase activity comprising an amino acid sequence as set forth in SEQ ID NO. 8 the provided polypeptide is selected from the group
a) a polypeptide comprising an amino acid sequence as set forth in SEQ ID NO. 8 or a sequence with at least 80% sequence identity to said sequence,
b) an isolated codon optimized nucleic acid fragment comprising a nucleotide sequence of at least 80% sequence identity to SEQ ID NO. 16
c) an isolated nucleic acid fragment comprising a sequence complementary to SEQ ID NO. 16,
d) an isolated nucleic acid fragment comprising a sequence which specifically hybridizes to said isolated nucleic acid fragment of b) or said complementary of c).

The term "nucleic acid molecule" is intended to indicate any single- or double stranded nucleic acid molecule comprising DNA (cDNA and/or genomic DNA), RNA (preferably mRNA), PNA, LNA and/or Morpholino.

As used herein the term "nucleic acid fragment" refers to an isolated nucleic acid molecule.

As used herein the term "gene" means a DNA sequence made up of nucleotides comprising a region (transcribed region), which is transcribed into an RNA molecule (e.g. directly into a mRNA without intron sequences) in a cell, operably linked to regulatory regions capable of regulating the expression of the polypeptide. A gene may thus comprise several operably linked sequences, such as untranslated regulatory regions (e.g. a promoter, enhancer, repressor), a 5' leader sequence comprising e.g. sequences involved in translation initiation, a (protein) coding region (cDNA or genomic DNA) and a 3' non-translated sequence comprising e.g. transcription termination sites.

As used herein the term "expression of a gene" or "gene expression" refers to the process wherein a DNA region (the coding region), which is operably linked to appropriate regulatory regions, particularly a promoter, is transcribed into an mRNA molecule. The mRNA molecule is then processed further (by post-transcriptional processes) within the cell, e.g. by translation initiation and translation into an amino acid chain (polypeptide), and translation termination by translation stop codons.

The term "complementary" as used herein refers to two nucleic acid molecules that can form specific interactions with one another. In the specific interactions, an adenine base within one strand of a nucleic acid can form two hydrogen bonds with thymine within a second nucleic acid strand when the two nucleic acid strands are in opposing polarities. Also in the specific interactions, a guanine base within one strand of a nucleic acid can form three hydrogen bonds with cytosine within a second nucleic acid strand when the two nucleic acid strands are in opposing polarities. Complementary nucleic acids as referred to herein, may further comprise modified bases wherein a modified adenine may form hydrogen bonds with a thymine or modified thymine, and a modified cytosine may form hydrogen bonds with a guanine or a modified guanine.

As used herein the term "specifically hybridize" refers to a reaction of the nucleic acid sequence in question in a hybridization solution containing 0.5 M sodium phosphate buffer, pH 7.2 containing 7% SDS, 1 mM EDTA and 100 mg/ml of salmon sperm DNA at 65°C for 16 hours and washing twice at 65°C for twenty minutes in a washing solution containing 9.5xSSC and 0.1% SDS.

As used herein the term "Wild type" (also written "wildtype" or "wild-type"), refers to a typical form of an enzyme or a gene as it most commonly occurs in nature.

The term "vector", as used herein, refers to a molecular vehicle used to transfer foreign genetic material into another cell. The vector itself is generally a DNA sequence that consists of an insert (sequence of interest) and a larger sequence that serves as the "backbone" of the vector. The purpose of a vector to transfer genetic information to another cell is typically to isolate, multiply, or express the insert in the target cell.

The term "plasmid", as used herein, refers to plasmid vectors, i.e. circular DNA sequence that are capable of autonomous replication within a suitable host due to an origin of replication ("ORI"). Furthermore, a plasmid may comprise a selectable marker to indicate the success of the transformation or other procedures meant to introduce foreign DNA into a cell and a multiple cloning site which includes multiple restriction enzyme consensus sites to enable the insertion of an insert. Plasmid vectors called cloning or donor vectors are used to ease the cloning and to amplify a sequence of interest. Plasmid vectors called expression or acceptor vectors are specifically for the expression of a gene of interest in a defined target cell. Those plasmid vectors generally show an expression cassette, consisting of a promoter, a ribosomal binding site, the transgene and a terminator sequence. For expression control those plasmids contains a repressor, which is localized on the plasmid backbone. Expression plasmids can be shuttle plasmids containing elements that enable the propagation and selection in different host cells.

As used herein the term "specifically hybridize" or "selectively hybridize" refers to a reaction of the nucleic acid sequence in question in a hybridization solution containing 0.5 M sodium phosphate buffer, pH 7.2 containing 7% SDS, 1 mM EDTA and 100 mg/ml of salmon sperm DNA at 65°C for 16 hours and washing twice at 65°C for twenty minutes in a washing solution containing 9.5 x SSC and 0.1% SDS.

The term "cDNA" means a DNA molecule that can be prepared by reverse transcription from a mature, spliced, mRNA molecule obtained from a eukaryotic or prokaryotic cell. cDNA lacks intron sequences that may be present in the corresponding genomic DNA. The initial, primary RNA transcript is a precursor to mRNA that is processed through a series of steps, including splicing, before appearing as mature spliced mRNA.

The term "coding sequence" means a nucleic acid sequence i.e. a polynucleotide, which directly specifies the amino acid sequence of a variant. The boundaries of the coding sequence are generally determined by an open reading frame, which begins with a start codon such as ATG, GTG or TTG and ends with a stop codon such as TAA, TAG, or TGA. The coding sequence may be a genomic DNA, cDNA, synthetic DNA, or a combination thereof.

In a further aspect what is described herein, relates to a polypeptide with carboxyesterhydrolase activity comprising the amino acid sequence of SEQ ID NO 2 or a sequence with at least 80% sequence identity.

Said polypeptide with carboxyesterhydrolase activity comprising the amino acid sequence of SEQ ID NO 2 or a sequence with at least 80% sequence identity can be characterized in that it is encoded by the polynucleotide of SEQ ID NO 10 or a polynucleotide sequence with at least 80% sequence identity to SEQ ID NO 10.

In a further aspect what is described herein, relates to a polypeptide with carboxyesterhydrolase activity comprising the amino acid sequence of SEQ ID NO 8 or a sequence with at least 80% sequence identity.

Said polypeptide with carboxyesterhydrolase activity comprising the amino acid sequence of SEQ ID NO 8 or a sequence with at least 80% sequence identity can be characterized in that it is encoded by the polynucleotide of SEQ ID NO 16 or a polynucleotide sequence with at least 80% sequence identity to SEQ ID NO 16.

In a further aspect what is described herein, relates to the use of a polypeptide with carboxyesterhydrolase activity comprising the amino acid sequence of SEQ ID NO 8 or a sequence with at least 80% sequence identity in a hydrolysis reaction.

In yet another aspect what is described herein, relates to an expression vector comprising at least one of the polynucleotides described above.

In yet another aspect what is described herein, relates to a host cell comprising at least one expression vector described above.

### Figure:

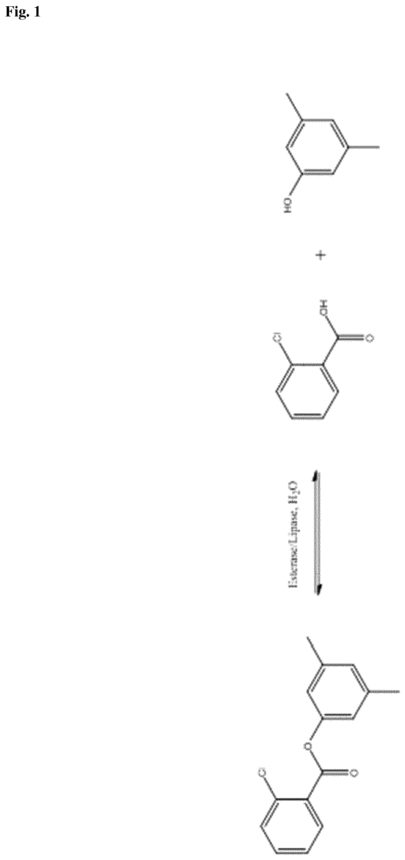

Fig. 1 depicts a schematic illustration of the hydrolysis reaction of 3,5-dimethylphenyl 2-chlorobenzoate to 2-chlorobenzoate and 3,5-dimethylphenole catalyzed by a carboxyesterhydrolase

### Examples:

### 1. Origin of the sequence information

The genome sequences were derived from *Alcanivorax borkumensis* SK2 ((Schneiker, V. a P. Martins dos Santos, et al. 2006) NCBI accession: NC_008260) and *Pseudomonas aestusnigri* VGXO14 U.S. Department of Energy Joint Genome Institute, JGI 850 Proj ID090574, accessed 02.09.2016 & NCBI accession: FNVE01000000.

### 2. Origin of the genetic material

The bacterium *Alcanivorax borkumensis SK-2* was first described in 1998 (Yakimov et al. 1998) and its complete genome sequence was published in 2006 (Schneiker, V. A. P. Martins dos Santos, et al. 2006). The organism *Alcanivorax borkumensis* was grown in shake flasks at 30°C and 130rpm in Marine Broth (DIFCO) using pyruvate as carbon source.

The organism *Pseudomonas aestusnigri* was isolated in 2011 and described in 2014 (Mulet et al. 2011; Sánchez et al. 2014). It was grown in shake flasks at 30°C and 130rpm in LB Medium.

Moreover, ester containing environmental samples were taken and enrichment cultures were established to select for organisms that can grow at 150rpm while using different esters that need to be hydrolyzed and one amid as carbon source. In case of niludipin and 1,4-dihydropyridine (dipropyl 2,6-dimethyl-4-phenyl-1,4-dihydropyridine-3,5-dicarboxylate) the cultures were grown at 37°C whereas the cultures using the other substrates were grown at 50°C. In order to do so shake flasks with 50mL (=20% of the filling volume) of M9 Mineral Medium and one of the carbon sources listed in Table 1 below were used. If growth could be detected 1mL of the culture was placed in new medium with the same ingredients. In the third enrichment round 40µg/mL cycloheximide were added to prevent eukaryotic growth.

**Table 1:**

| **Substrate** | **Available carbon soruce** | **MW** | **g/L** |
|---|---|---|---|
| Daminozid | Succinate | 160 | 5 |
| (1R)-(-)-Dimenthyl succinate | Succinate | 394 | 15 |
| cyclohexanol succinat | Succinate | 200 | 10 |
| dipropyl 2,6-dimethyl-4-phenyl-1,4-dihydropyridine-3,5-dicarboxylate | Propanol | 357,45 | 15 |
| Niludipine | 2-(Propyloxy)ethanol | 490 | 20 |

The extraction of the genetic material from the cell mass was carried out using a conventional phenolchloroform extraction followed by isopropyl precipitation.

### 3. Identification of active carboxyesterhydrolases

Genome and Metagenome libraries were established using the method of Nacke et al. 2011. In order to do so the TOPO® XL PCR Cloning Kit (Thermo Fisher Scientific Inc.) which included the vector for DNA replication (pCR-XL-TOPO®) as well as the host cell (Escherichia coli TOP10®) for activity based screening was used. If cells showed activity towards tributyrin (Kok et al. 1993) the plasmid DNA was isolated using the innuPREP Plasmid Mini Kit (Analytik Jena AG). A DNA sequence of around 100 to 800 nucleotides of the flanking regions of the genomic or metagenomics DNA-fragment was determined using the oligonucleotides of the TOPO® XL PCR Cloning Kit (Thermo Fisher Scientific Inc.). The size of the DNA fragments was determined via agarose gel electrophoresis after hydrolysis by the restriction endonuclease EcoRI (Thermo Fisher Scientific Inc.) In the case of DNA fragments from known organism the DNA fragment was identified via comparison with the known genome and due to the previously determined size of the fragment. If no genome or metagenome library was available the whole sequence was determined.

The determined sequences of the DNA fragments were analyzed for bacterial genes and annotated (ORFfinder, https://www.ncbi.nlm.nih.gov/orffinder/; BASYS, (Van Domselaar et al. 2005)). Genes which seemed to be involved in hydrolysis reactions (e.g. esterases, carboxylesterases, lipases, α/β-hydrolases) were amplified using specific oligonucleotides as (data not shown) and cloned into the expression vector pET-22b(+) (Merck Chemicals GmbH, Sambrook et al. 1989; Jeong et al. 2012).

In order to do so the 5,3 kB pair fragment of the pET-22b(+) Vector was used, which was generated via hydrolysis with the restriction endonucleases NdeI and XhoI (Thermo Fisher Scientific Inc.) and isolated via preparative Agarose electrophoresis. Only for Paes PE-H the restriction endonuclease *Xba*I was used instead of *Nde*I. Insertion of the genes into the vector was such that they were in reading direction of the T7-RNA polymerase specific promoter as well as in frame with the six histidine residue coding region of the vector backbone. The stop codon was thereby removed.

Moreover, genes were also amplified and cloned as described above based on the above described annotation of the genomes as well as an additional annotation using the BASYS program (Van Domselaar et al. 2005). Hydrolytic activity of the recombinant E. coli clones was demonstrated with the Substrate Tributyrin.

The recombinant expression vectors were inserted into the heterologous host Escherichia coli BL21(DE3) and expressed. In this host the heterologous genes are transcribed by the T7 promoter, which is specifically recognized by the chromosomal coded RNA Polymerase of bacteriophage T7. The Expression of the RNA polymerase can be induced via addition of an inducer. For the expression the transformed E. coli BL21(DE3) cells were cultured for 16h at 37°C. With a part of the resulting culture, another culture was subsequently inoculated, which was grown only in the presence of lactose as inducer of the heterologous gene expression (as described previously (https://openwetware.org/wiki/Lidstrom:Autoinduction_Media, "Simplifed version from Imperial College"). Following 20h of culture on a shaking platform, the cells were harvested by centrifugation, lysed and forwarded to assessment of esterase activity and/or purification.

Identified enzymes were purified using the protocol "Protocol 12. Batch purification of 6xHis-tagged proteins from E. coli under native conditions" (Qiagen 2003) and the Ni-NTA Superflow resin (Qiagen) as stationary chromatography phase.

Enzymatic activity of the produced heterologous proteins was verified using the substrate 4-Nitrophenylbutyrate. Lysate of E. coli BL21(DE3) transformed with an empty pET-22b(+) vector, which did not comprise a gene encoding for an carboxyesterhydrolase, was used as control. If Nitrophenylbutyrate is cleaved by an carboxyesterhydrolase, one of the products is 4-Nitrophenol, which can be photometrical quantified at a wavelength of 410 nm. It was assumed that enzymes which lead to a higher absorption than controls were functional carboxyesterhydrolases.

The inventors of the present invention determined for the first time that the claimed enzymes can be used for the enzymatic conversion of the sterically demanding reaction of 3,5-dimethylphenyl 2-chlorobenzoate to 3,5-dimethylphenole and 2-chlorobenzoic acid (see Fig. 1). Progression of said reaction can be monitored due to the acidification of the reaction mixture via monitoring the pH value, analogous to the method described in (Peña-García et al. 2016). Therefore, an particularly suited assay was developed using the pH indicator dye nitrazine yellow in weakly phosphate buffer reaction mixture, whereby the release of the acid (2-chlorobenzoic acid) becomes visible by the change of the dye color from blue at pH 7 to yellow at pH below 7 . This can be determined spectrophotometrically by an increase of the ratio of the absorptions at 450 nm and 600 nm. The correlation of the color shift and free 2-chlorobenzoic acid was confirmed by HPLC analysis. Either purified enzymes or cells of the expression cultures described above were used for the reactions. Lysis of the cells was carried out mechanically using sonication (Bandelin Sonopuls HD 2070, Mikrospitze MS73, 6 min, Cycle 5, 30 % Power, on ice) in 10 mM potassium phosphate buffer at pH 7,2. 100 µl of lysed cells or enzyme solution were mixed with 900 µl substrate (1 mM potassium phosphate buffer at pH 7, 10 mM 3,5-dimethylphenyl 2-chlorobenzoate; 5 % acetonitrile; 20 µg/ml nitrazine yellow) and incubated at 30 °C for at least 18 hours. Afterwards, acidification was detected via the change of indicator color as stated above after transfer of a 200µL aliquot of the reaction mixture to a microtiter plate using a Tecan infinite M1000Pro photometer.

Lysat of E. coli BL21(DE3) transformed with an empty pET-22b(+) vector, which did not comprise a gene encoding for an carboxyesterhydrolase, but otherwise was treated identically was used as control. In the case of enzyme solutions, the buffer was used without enzyme.

Surprisingly, it was found that all enzymes listed in Table 3 showed hydrolytic activity towards 3,5-dimethylphenyl 2-chlorobenzoate. A cleavage of this substrate by carboxyesterhydrolases has not been described so far.

**Table 3: activity of the novel carboxyesterhydrolases with 3,5-dimethylphenyle 2-chlorobenzoate**

| **Enzyme** | **Activity [A 450 / 600]** |
|---|---|
| SEQ ID No 1 | 1,35 |
| SEQ ID No 2 | 0,5 |
| SEQ ID No 3 | 0,36 |
| SEQ ID No 4 | 0,38 |
| SEQ ID No 5 | 0,92 |
| SEQ ID No 6 | 0,56 |
| SEQ ID No 7 | 0,15 |
| SEQ ID No 8 | 0,36 |

## Claims

1. A polypeptide selected from the group consisting of SEQ ID NO 1 to SEQ ID NO 8 or a sequence with at least 80% sequence identity to any one of said sequences, wherein said polypeptide has activity towards 3,5-dimethylphenyl 2-chlorobenzoate.

2. A polypeptide according to claim 1 wherein said polypeptide has at least 90% sequence identity to any one of the sequences selected from the group consisting of SEQ ID NO 1 to SEQ ID NO 8.

3. Use of a polypeptide according to claims 1 or 2 for the enzymatic conversion of 3,5-dimethylphenyl 2-chlorobenzoate to 3,5-dimethylphenol and 2-chlorobenzoic acid.

4. Polypeptide with an activity according to claims 1 or 2, **characterized in that**
a) the polypeptide of SEQ ID NO 1 is encoded by the nucleic acid sequence of SEQ ID NO 9 or a nucleic acid sequence with at least 80% sequence identity to SEQ ID NO 9 and
b) the polypeptide of SEQ ID NO 2 is encoded by the nucleic acid sequence of SEQ ID NO 10 or a nucleic acid sequence with at least 80% sequence identity to SEQ ID NO 10 and
c) the polypeptide of SEQ ID NO 3 is encoded by nucleic acid sequence of SEQ ID NO 11 or a nucleic acid sequence with at least 80% sequence identity to SEQ ID NO 11 and
d) the polypeptide of SEQ ID NO 4 is encoded by the nucleic acid sequence of SEQ ID NO 12 or a nucleic acid sequence with at least 80% sequence identity to SEQ ID NO 12 and
e) the polypeptide of SEQ ID NO 5 is encoded by the nucleic acid sequence of SEQ ID NO 13 or a nucleic acid sequence with at least 80% sequence identity to SEQ ID NO 13 and
f) the polypeptide of SEQ ID NO 6 is encoded by the nucleic acid sequence of SEQ ID NO 14 or a nucleic acid sequence with at least 80% sequence identity to SEQ ID NO 14 and
g) the polypeptide of SEQ ID NO 7 is encoded by the nucleic acid sequence of SEQ ID NO 15 or a nucleic acid sequence with at least 80% sequence identity to SEQ ID NO 15 and
h) the polypeptide of SEQ ID NO 8 is encoded by the nucleic acid sequence of SEQ ID NO 16 or a nucleic acid sequence with at least 80% sequence identity to SEQ ID NO 16.

5. A polypeptide with carboxyesterhydrolase activity comprising the amino acid sequence of SEQ ID NO 8 or a sequence with at least 80% sequence identity.

6. Method for using a polypeptide of claim 1, for generating 2-chlorobenzoate and 3,5-dimethylphenole from 3,5-dimethylphenyl 2-chlorobenzoate under suitable conditions.

7. Expression vector comprising at least one of the nucleic acid sequences of claim 4.

8. Host cell comprising at least one expression vector according to claim 7.


SEQUENCE LISTING

<110>  Bayer Aktiengesellschaft / CISC

<120>  Novel lipases

<130>  BTS173011

<160>  16

<170>  PatentIn version 3.5

<210>  1
<211>  296
<212>  PRT
<213>  Alcanovorax borkumii

<400>  1

Met Ser Leu Gln Ala Arg Leu Ile Lys Ala Val Thr Lys Arg Thr Ile
1               5                   10                  15

Lys Arg Ser Gly Leu Asn Gln Asp Gln Leu Val Arg His Leu Arg Lys
            20                  25                  30

Val Phe Asn Glu Thr Pro Val Leu Thr Leu Leu Pro Arg Gly Val Lys
        35                  40                  45

Leu Ser Arg Val Glu His Pro Ala Phe Thr Gly Asp Arg Ile Ser Val
    50                  55                  60

Gln Arg Pro Glu Met Ala Val Leu Tyr Leu His Gly Gly Ala Tyr Ile
65                  70                  75                  80

Gly Gly Ile Thr Lys Thr Tyr His Asn Leu Ala Gly Arg Leu Ala Lys
                85                  90                  95

Lys Leu Asn Ala Glu Val Phe Leu Pro Val Tyr Pro Phe Ala Pro Glu
            100                 105                 110

His Pro Tyr Pro Ala Ala Val Asn Arg Val Met Glu Ala Tyr Glu Tyr
        115                 120                 125

Leu Leu Ser Leu Gly Lys Gln Pro Gln Asp Ile Val Ile Ala Gly Asp
    130                 135                 140

Ser Ala Gly Gly Gly Leu Thr Leu Ala Thr Leu Leu His Ile Arg Asp
145                 150                 155                 160

Lys Gly Leu Glu Gln Pro Arg Cys Ala Val Thr Phe Ser Pro Ala Ser
                165                 170                 175

Asn Ala Phe Pro Asp Asp Ser Ile Leu Glu Ala Leu Asp Pro Ser Asp
            180                 185                 190

Ala Met Leu Ser Ala Asp Ile Ile Arg Thr Ala Ile Glu Ile Tyr Thr
        195                 200                 205

Pro Asn Pro Glu Asp Arg Ser Gln Pro Tyr Ala Ser Pro Cys Leu Gly
    210                 215                 220

Asp Tyr Thr Asn Ile Cys Pro Leu Leu Ile Thr Ala Ser Thr Asp Glu
225                 230                 235                 240

Leu Leu Tyr Ala Asp Gly Lys Arg Val Lys Arg Val Ala Glu Lys Ala
                245                 250                 255

Gly Val Lys Val Thr Trp Ile Glu Arg Pro Gly Val Phe His Val Trp
            260                 265                 270

Pro Val Met Val Pro Phe Leu Pro Glu Ala Asn Lys Asp Leu Lys Arg
        275                 280                 285

Ile Val Ala Phe Ile Lys Glu Ala
    290                 295

<210>  2
<211>  368
<212>  PRT
<213>  Artificial Sequence

<220>
<223>  Cyc TB #025

<400>  2

Met Thr Thr Thr Ser Asn Ser Thr Glu Thr Gly Arg Arg Pro Gly Arg
1               5                   10                  15

Leu Gly Asp Pro Asp Arg Cys Leu Arg Thr Asp Pro Arg Thr Asp Pro
            20                  25                  30

Arg Thr Val Glu Ala Leu Ala Pro Phe Gly Leu Asp Val Asn Ala Ala
        35                  40                  45

Pro Ala Pro Ile Gly Pro Asp Ala Pro Arg Glu Gln Gln Leu Glu Tyr
    50                  55                  60

Ala Met Gly Ala Glu Ala Ala Phe Glu Gly Val Phe Ala Ala Leu Met
65                  70                  75                  80

Asp Gly Leu Asp Pro Val Pro Gly Ile Glu Arg Arg Thr Glu Thr Ile

                85                  90                  95

Ser Gly Pro Ala Gly Asn Glu Ile Lys Leu Tyr Val His Arg Pro Ala
            100                 105                 110

Gly Ala Val Gly Pro Leu Pro Gly Ile Phe His Ile His Gly Gly Gly
        115                 120                 125

Met Val Ile Leu Gln Ala Ala Gly Pro Val Tyr Val Arg Phe Arg Asp
    130                 135                 140

Glu Leu Ala Ala Thr Gly Thr Val Val Val Gly Val Glu Tyr Arg Asn
145                 150                 155                 160

Gly Ala Gly Val Leu Gly Pro His Pro Phe Pro Ala Gly Leu His Asp
                165                 170                 175

Cys Ala Val Ala Leu Asp Trp Val His Ala Arg Arg Ala Glu Leu Gly
            180                 185                 190

Ile Ser Thr Leu Thr Val Ala Gly Glu Ser Gly Gly Gly Asn Leu Thr
        195                 200                 205

Leu Ala Thr Ala Ile Arg Ala Lys Arg Glu Gly Arg Leu Asp Ala Ile
    210                 215                 220

Asp Gly Val Tyr Ala Leu Val Pro Tyr Ile Ser Gly Met Tyr Gly Arg
225                 230                 235                 240

Ser Arg Glu Glu Arg Glu Ala Glu Leu Pro Ser Leu Val Glu Cys Asp
                245                 250                 255

Gly Tyr Phe Ile Ser Cys Asp Leu Cys Ala Val Phe Val Glu Val Tyr
            260                 265                 270

Asp Pro Gly Thr Ala His Leu Thr Asp Pro Leu Ala Trp Pro Tyr His
        275                 280                 285

Ala Ala Arg Glu Asp Leu Val Gly Leu Pro Pro His Val Ile Ser Val
    290                 295                 300

Asn Glu Val Asp Pro Leu Arg Asp Glu Gly Leu Ala Tyr Tyr Arg Lys
305                 310                 315                 320

Leu Val Glu Ala Gly Val Glu Ala Arg Ser Arg Val Val Pro Gly Ala
                325                 330                 335

Cys His Ala Ala Asp Met Met Phe Arg Lys Ala Ala Pro Asp Met Tyr
            340                 345                 350

Glu Ala Thr Val Gln Asp Ile His Asp Phe Val Thr Ser Leu His Arg
        355                 360                 365

<210>  3
<211>  552
<212>  PRT
<213>  Pseudomonas aestusnigr

<400>  3

Met Ser Thr Ala Arg Leu Thr Pro Thr Thr Ala Ser Thr Leu Lys Leu
1               5                   10                  15

Leu Ser Val Ala Val Ala Ala Leu Thr Leu Ser Ala Cys Leu Ser Gly
            20                  25                  30

Gly Gly Gly Gly Arg Ala Asp Gly Pro Asn Pro Leu Ala Ile Glu Thr
        35                  40                  45

Ser Glu Gly Lys Val Val Gly Ile Ser Asn Asp Gly Ile Arg Val Phe
    50                  55                  60

Arg Gly Ile Pro Tyr Ala Ala Pro Pro Val Gly Asp Leu Arg Leu Ala
65                  70                  75                  80

Pro Pro Gln Pro Pro Ala Ser Arg Ser Glu Thr Leu Arg Leu Ser Glu
                85                  90                  95

Glu Phe Gly Asn Ser Cys Pro Gln Ser Asp Leu Thr Thr Gly Gln Gln
            100                 105                 110

Val Gly Asn Glu Asp Cys Leu Tyr Leu Asn Val Tyr Ala Pro Ala Glu
        115                 120                 125

Ala Glu Asp Leu Pro Val Met Val Trp Ile His Gly Gly Ala Phe Val
    130                 135                 140

Phe Gly Asn Gly Gly Gly Glu Tyr Asp Pro Thr Arg Leu Val Glu Gln
145                 150                 155                 160

Asp Val Ile Val Val Thr Leu Asn Tyr Arg Leu Gly Asn Leu Gly Phe
                165                 170                 175

Leu Ala His Pro Ala Leu Glu Ser Asp Ala Gly Asn Phe Ala Leu Met
            180                 185                 190

Asp Gln Gln Leu Ala Leu Ala Trp Val Lys Glu Asn Ile Ala Ala Phe
        195                 200                 205

Gly Gly Asp Pro Ala Asn Val Thr Ile Phe Gly Glu Ser Ala Gly Gly
    210                 215                 220

His Ser Val Met Ser His Ile Val Ser Pro Arg Ala Glu Glu Ala Asp
225                 230                 235                 240

Leu Phe Gln Arg Ala Ile Val Gln Ser Gly Ser Tyr Ala Pro Phe Gln
                245                 250                 255

Met Pro Lys Ala Thr Ala Gln Phe Leu Gly Thr Ser Val Ala Asn Gly
            260                 265                 270

Leu Gly Cys Thr Asp Pro Glu Thr Ala Ala Ser Cys Leu Arg Ser Leu
        275                 280                 285

Pro Val Ser Ala Phe Leu Ala Ala Gln Gly Ser Gln Ser Ile Pro Val
    290                 295                 300

Val Asp Pro Asp Asp Asp Leu Leu Pro Lys Ser Ile Gln Gln Ala Leu
305                 310                 315                 320

Ala Asp Gly Asp Phe Asn Ser Ser Leu Asp Ile Met Ile Gly Ser Asn
                325                 330                 335

Gln Asn Glu Gly Thr Leu Phe Val Ala Leu Asp Glu Val Gly Gly Asp
            340                 345                 350

Pro Ile Asp Asp Glu Ala Glu Tyr Arg Glu Arg Val Ala Glu Phe Phe
        355                 360                 365

Gln Pro Tyr Gln Ala Ser Ile Pro Phe Asp Asp Asp Gln Ile Ala Thr
    370                 375                 380

Asp Tyr Leu Asp Phe Val Asp Gly Ala Ala Lys Pro Phe Glu Ala Ala
385                 390                 395                 400

Leu Ser Gly Ile Trp Thr Asp Phe Met Phe Ala Cys Asn Ala Tyr Ser
                405                 410                 415

Gln Ala Ser Thr Phe Ala Gly Ala Ser Met Asn Thr Phe Gln Tyr Trp
            420                 425                 430

Phe Arg Asp Glu Asp Ala Pro Trp Thr Leu Val Pro Pro Phe Ala Val
        435                 440                 445

Ser Phe Pro Leu Gly Ala Thr His Ala Gly Glu Ile Pro Tyr Val Leu
    450                 455                 460

Tyr Pro Gln Ala Ile Met Glu Gln Arg Tyr Thr Gly Asp Pro Asp Asp
465                 470                 475                 480

Leu Asn Ser Leu Ala Gly Glu Met Val Asp Tyr Trp Thr Gln Phe Ala
                485                 490                 495

Lys Thr Gly Asp Pro Asn Thr Thr Asp Gly Val Ala Ala Ala Trp Gln
            500                 505                 510

Gln Ala Ala Thr Gly Asn Leu Leu Thr Leu Asp Val Pro Asn Ala Ser
        515                 520                 525

Asn Ala Asn Thr Leu Gly Phe Leu Gly Tyr His His Cys Ser Tyr Trp
    530                 535                 540

Ala Asp Pro Pro Leu Val Leu Pro
545                 550

<210>  4
<211>  405
<212>  PRT
<213>  Pseudomonas aestusnigr

<400>  4

Met Ala Asn Ser Asn Cys Ile Ala Gly Val Asp Leu Glu Arg Leu Ser
1               5                   10                  15

Arg Ile Gly Thr His Leu Gln Ser Ala Tyr Ile Asp Ala Gly Lys Leu
            20                  25                  30

Pro Gly Ala Leu Thr Leu Val Ala Arg Arg Gly Gln Val Val Tyr Cys
        35                  40                  45

Glu Ala Gln Gly Leu Arg Asp Val Glu Arg Gly Leu Pro Val Glu Arg
    50                  55                  60

Asp Thr Leu Phe Arg Ile Tyr Ser Met Thr Lys Pro Val Thr Ser Val
65                  70                  75                  80

Ala Leu Met Gln Leu Tyr Glu Gln Gly Arg Phe Leu Leu Asp Asp Pro
                85                  90                  95

Val His Lys Tyr Ile Pro Ser Trp Ser Asn Leu Arg Val Tyr Lys Thr
            100                 105                 110

Gly Ser His Pro Gln Ile Leu Thr Thr Pro Cys Glu Arg Pro Met Thr
        115                 120                 125

Ile Arg Asp Leu Leu Thr His Gln Ser Gly Leu Thr Tyr Gly Phe Met
    130                 135                 140

Asn Arg Thr Asn Val Asp Ala Ala Tyr Arg Ala Leu Lys Leu Asp Gly
145                 150                 155                 160

Gly Pro Gly Leu Thr Leu Asp Arg Leu Ile Asp Glu Leu Ser Arg Leu
                165                 170                 175

Pro Leu Glu Phe Ser Pro Gly Thr Ala Trp Asn Tyr Ser Val Ala Thr
            180                 185                 190

Asp Val Cys Gly Tyr Leu Val Gln Val Leu Ser Gly Met Ser Leu Asp
        195                 200                 205

Asp Tyr Phe Lys Arg His Ile Phe Asp Pro Leu Glu Met Ser Asp Thr
    210                 215                 220

Phe Phe Thr Val Pro Ala Asp Arg Ile Pro Arg Phe Ala Ala Cys Tyr
225                 230                 235                 240

Gln Phe Gln Pro Gly Gly Ser Phe Ser Leu Gln Asp Asp Pro Gln Asp
                245                 250                 255

Ser Ser Phe Thr Lys Ala His Gly Phe Leu Ser Gly Gly Gly Gly Leu
            260                 265                 270

Val Ser Thr Ile Asp Asp Tyr Tyr Arg Phe Ala Gln Ala Leu Ala Asn
        275                 280                 285

Gly Gly Glu Leu Asn Gly Ala Arg Ile Ile Gly Arg Lys Thr Leu Glu
    290                 295                 300

Phe Met Arg Met Asn His Leu Pro Gly Asn Gln Asp Leu Pro Gly Val
305                 310                 315                 320

Ser Val Gly Ser Phe Ser Glu Thr Pro Tyr Glu Gly Ser Gly Phe Gly
                325                 330                 335

Leu Gly Phe Ser Val Lys Ile Asp Val Ala Lys Ser Gln Thr Asn Gly
            340                 345                 350

Ser Val Gly Glu Tyr Gly Trp Gly Gly Met Ala Ser Thr Asn Phe Phe
        355                 360                 365

Val Asp Pro Val Glu Asp Leu Leu Met Val Phe Met Thr Gln Leu Ile
    370                 375                 380

Pro Ser Ser Ser Tyr Pro Val Arg Gln Glu Leu Arg Ala Ile Ile Asn
385                 390                 395                 400

Gly Ala Leu Val Asp
                405

<210>  5
<211>  304
<212>  PRT
<213>  Pseudomonas aestusnigr

<400>  5

Met Pro Phe Asn Lys Lys Ser Val Leu Ala Leu Cys Gly Ala Gly Ala
1               5                   10                  15

Leu Leu Phe Ser Met Ser Ala Leu Ala Asn Asn Pro Ala Pro Thr Asp
            20                  25                  30

Pro Gly Asp Ser Gly Gly Gly Ser Ala Tyr Gln Arg Gly Pro Asp Pro
        35                  40                  45

Ser Val Ser Phe Leu Glu Ala Asp Arg Gly Gln Tyr Ser Val Arg Ser
    50                  55                  60

Ser Arg Val Ser Ser Leu Val Ser Gly Phe Gly Gly Gly Thr Ile Tyr
65                  70                  75                  80

Tyr Pro Thr Gly Thr Thr Gly Thr Met Gly Ala Val Val Val Ile Pro
                85                  90                  95

Gly Phe Val Ser Ala Glu Ser Ser Ile Asp Trp Trp Gly Pro Lys Leu
            100                 105                 110

Ala Ser Tyr Gly Phe Val Val Met Thr Ile Asp Thr Asn Thr Gly Phe
        115                 120                 125

Asp Gln Pro Pro Ser Arg Ala Arg Gln Ile Asn Asn Ala Leu Asp Tyr
    130                 135                 140

Leu Val Ser Gln Asn Ser Arg Ser Ser Ser Pro Val Arg Gly Met Ile
145                 150                 155                 160

Asp Thr Asn Arg Leu Gly Val Ile Gly Trp Ser Met Gly Gly Gly Gly
                165                 170                 175

Thr Leu Arg Val Ala Ser Glu Gly Arg Ile Lys Ala Ala Ile Pro Leu
            180                 185                 190

Ala Pro Trp Asp Thr Thr Ser Tyr Tyr Ala Ser Arg Ser Gln Ala Pro
        195                 200                 205

Thr Leu Ile Phe Ala Cys Glu Ser Asp Val Ile Ala Pro Val Leu Gln
    210                 215                 220

His Ala Ser Pro Phe Tyr Asn Ser Leu Pro Ser Ser Ile Asp Lys Ala
225                 230                 235                 240

Phe Val Glu Ile Asn Gly Gly Ser His Tyr Cys Gly Asn Gly Gly Ser
                245                 250                 255

Ile Tyr Asn Asp Val Leu Ser Arg Phe Gly Val Ser Trp Met Lys Leu
            260                 265                 270

His Leu Asp Glu Asp Ser Arg Tyr Lys Gln Phe Leu Cys Gly Pro Asn
        275                 280                 285

His Thr Ser Asp Ser Gln Ile Ser Asp Tyr Arg Gly Asn Cys Pro Tyr
    290                 295                 300

<210> 6
<211> 218
<212> PRT
<213> Pseudomonas aestusnigr

<400> 6

Met Ser Pro His Ile Ile Glu Pro Ala Thr Pro Ala Thr Arg Ser Val
1               5                   10                  15

Ile Trp Leu His Gly Leu Gly Ala Asp Cys Tyr Asp Phe Val Pro Val
            20                  25                  30

Val Glu Ala Leu Gln Leu Pro Ala Asp His Ala Ile Arg Phe Ile Phe
        35                  40                  45

Pro Gln Ala Pro Thr Arg Pro Val Thr Ile Asn Gly Gly Phe Pro Met
    50                  55                  60

Pro Cys Trp Tyr Asp Ile Leu Gly Met Ser Pro Ala Arg Ala Ile Asn
65                  70                  75                  80

Gln Ser His Leu Asp Glu Ala Val Ala Leu Val Arg Gln Leu Ile Asp
                85                  90                  95

Glu Gln Cys Ala Gln Gly Ile Ala Leu Glu Asn Ile Ile Leu Ala Gly
            100                 105                 110

Phe Ser Gln Gly Gly Ala Val Val Leu Cys Thr Ala Val Ser Ser Glu
        115                 120                 125

Leu Pro Leu Gly Gly Val Met Ala Leu Ser Thr Tyr Gly Pro Gly Leu
    130                 135                 140

Asp Leu Leu Leu Glu Gln His Pro Ala Arg Gln Pro Leu Glu Leu Phe
145                 150                 155                 160

Cys Ala His Gly Arg Phe Asp Asp Val Leu Pro Leu Ala Met Gly Arg
                165                 170                 175

Glu Ala His Asp Leu Met Gln Ala Ala Gly His Gln Thr Arg Trp Tyr
            180                 185                 190

Glu Tyr Pro Met Ala His Glu Val Cys Met Asp Glu Ile Ala Asp Ile
        195                 200                 205

Arg Arg Trp Leu Val Glu Arg Leu Gly Leu
    210                 215

<210> 7
<211> 309
<212> PRT
<213> Artificial Sequence

<220>
<223> Dim004

<400> 7

Met Ser Pro Pro Gln Arg Leu Arg Ala Gly Leu Leu Arg Gly Cys Leu
1               5                   10                  15

Arg Leu Leu Phe Arg Gly Leu Val Arg Pro Pro Met Pro Val Ala Leu
            20                  25                  30

Gln Arg Gly Val Leu Arg Gly Leu Ser Leu Ala Thr Leu Gly Ala Gly
        35                  40                  45

Gly Val Val Arg Glu Arg Arg Ser Leu Ala Gly Val Pro Cys Glu Trp
    50                  55                  60

Leu Arg Pro Arg Gly Val Glu Ala Arg Arg Arg Val Leu Tyr Leu His
65                  70                  75                  80

Gly Gly Ala Tyr Leu Ile Gly Ser Pro Ala Thr His Arg Ala Ile Thr
                85                  90                  95

Thr His Leu Ala Arg Arg Cys Ala Ala Glu Val Cys Ala Val Asp Tyr
            100                 105                 110

Arg Arg Ala Pro Glu His Pro Phe Pro Ala Ala Arg Asp Asp Ala Leu
        115                 120                 125

Ala Val Tyr Leu Ala Leu Leu Glu Ala Gly His Ser Pro Arg Arg Leu
    130                 135                 140

Leu Leu Ala Gly Asp Ser Ala Gly Gly His Leu Ala Leu Ser Leu Ala
145                 150                 155                 160

Leu Glu Leu Lys Ala Arg Gly Leu Pro Leu Pro Ala Gly Leu Leu Leu
                165                 170                 175

Phe Ser Pro Trp Thr Asp Leu Gly Cys Gln Arg Leu His Thr Pro Ala
            180                 185                 190

Ala Gly Asp Pro Leu Leu Ser Arg Ala Trp Leu Glu Ser Ala Val Arg
        195                 200                 205

Leu Leu Leu Pro Ala Asp Ala Glu Pro Gly Ser Ala Ala Leu Ser Pro
    210                 215                 220

Leu His Ala Asp Leu Ala Gly Leu Pro Pro Leu Leu Val Gln Val Gly
225                 230                 235                 240

Glu Asp Glu Leu Leu Arg Asp Asp Ser Leu Arg Leu Ala Gln Arg Ala
                245                 250                 255

Gly Glu Gln Gly Val Ala Val Arg Leu Gln Arg Tyr Ala Gly Cys Trp
            260                 265                 270

His Val Phe Gln Ala His Ala Gly Val Leu Ala Ser Ala Asp Arg Ala
        275                 280                 285

Leu Asp Glu Ala Ala Ala Phe Val Val Glu Cys Ser Ala Glu Gly Asp
    290                 295                 300

Arg Cys Arg Thr Ser
305

<210>  8
<211>  349

<212>  PRT
<213>  Artificial Sequence

<220>
<223>  1,4D #003

<400>  8

Met Phe Asp Pro Ala Arg Asn Arg Lys Leu Tyr Phe Gly His Val Phe
1               5                   10                  15

Leu Ala Arg Thr Leu Gly Arg Ala Pro Pro Gly Gly Pro Val Leu Ile
            20                  25                  30

Ala Arg Ile Leu Arg Leu Ala Thr Leu Ala Gly Ala Leu Gly Val Leu
        35                  40                  45

Gly Leu Ala Ala Val Leu Ala Leu Ala Thr Pro Leu Gly Trp Pro Gly
    50                  55                  60

Ala Leu Ala Cys Ser Ile Gly Val Val Pro Ala Gly Phe Ala Ile Val
65                  70                  75                  80

Leu Gly Val Glu Phe Leu Ala Ala Ala Leu Leu Gly Asp Arg His Trp
                85                  90                  95

Arg Gly Leu Val Arg Ala Trp Ala Gly Glu Val Leu Ala Ser Met Arg
            100                 105                 110

Thr Phe Leu Leu Ala Val Pro Trp Leu Gly Asp Arg Glu Val Ala Ser
        115                 120                 125

Thr Ala Gly Asp Arg Leu Pro Val Val Leu Val His Gly Tyr Phe Cys
    130                 135                 140

Asn Arg Ala Val Trp Arg Pro Phe Ala Arg Arg Leu Ala Glu Ala Gly
145                 150                 155                 160

His Pro Val Thr Ala Val Ser Leu Asp Pro Pro Phe Gly Pro Ile Asp
                165                 170                 175

Ala His Ala Ala Thr Ile Glu Thr Ala Val Ala Arg Ala Gly His Gly
            180                 185                 190

Arg Gly Pro Val Val Leu Val Gly His Ser Met Gly Gly Leu Ala Ile
        195                 200                 205

Arg Ala Phe Leu Arg Arg Ala Gly Ser Asp Gly Ile Ala Gly Val Val
    210                 215                 220

Thr Leu Gly Ser Pro His Arg Gly Thr Arg Met Ala Ala Val Gly Arg
225                 230                 235                 240

Ser Pro Ala Ala Arg Glu Met Arg Arg Gly Ser Ala Trp Leu Glu Ala
                245                 250                 255

Leu Ala Ala His Glu Ala Gln Gln Arg Glu Ala Ser Gly Asp Ala Ala
            260                 265                 270

Ala Pro Pro Leu Cys Ile Ile Leu Thr Leu Asn Asp Asn Ile Val Tyr
        275                 280                 285

Pro Gln Gly Glu Gln Val Leu Pro Gly Ala Arg Val Leu Arg Leu Asp
    290                 295                 300

Gly Val Gly His Leu Asp Leu Val Tyr Arg Pro Ser Val Gly Arg Leu
305                 310                 315                 320

Val Glu Ala Glu Ile Ala Arg Leu Glu Ala Gly Ile Thr Ala Ala Gly
                325                 330                 335

Ala Arg Ala Ala Asn Gly Lys Leu Ala Ala Ala Leu Glu
            340                 345

<210>  9
<211>  891
<212>  DNA
<213>  Alcanovorax borkumii

<400>  9
atgagccttc aagcccgcct catcaaagcc gtgaccaagc gcaccatcaa acgctccggt      60

ctcaaccaag accaactagt acggcatctg cgtaaggtat tcaacgaaac ccctgttctg     120

accctattac cccgtggagt aaaactcagt cgcgtggagc accccgcttt taccggtgat     180

cgaatcagcg tgcagcgtcc agaaatggcg gtgctttacc tgcatggcgg cgcctacatt     240

ggcggcatca ccaagaccta ccacaactta gccgggcggt tggcaaaaaa attaaacgct     300

gaggtatttt tacccgtcta tccgtttgcc ccagagcatc cttacccggc cgcagtgaat     360

cgagtcatgg aggcctacga atacctatta agcctaggca agcaaccgca ggatatcgtt     420

attgccggcg attccgccgg tggtgggctc accttggcca cgctgctgca tatccgcgac     480

aaaggcctgg aacaaccccg ctgcgcggtg acattttcgc ccgccagcaa cgcctttcct     540

gacgacagta tcctggaggc cctagacccc agcgatgcga tgctgtctgc agacatcatt     600

cgcactgcta tcgagatcta tacccccaac ccagaagacc gcagccagcc ctacgcctcc     660

ccctgcctag gagactacac caacatctgc ccgctactga tcaccgccag caccgacgaa     720

ctgctttatg cggatggcaa gcgagtgaag cgcgtggcgg aaaaggcggg cgtcaaggtg 780

acatggatag agcgccccgg cgtattccat gtatggccgg tcatggtgcc cttcctgcca 840

gaggcgaaca aagacctgaa acggattgtc gcattcatta aagaagcata g 891

<210> 10
<211> 1107
<212> DNA
<213> Artificial Sequence

<220>
<223> Cyc TB#025

<400> 10
atgacgacga cgtcgaactc caccgagacc ggacggcgcc ccggacggct gggcgatccg 60

gaccgctgcc tgcgcaccga tccccgcacc gatccccgca ccgtcgaggc actggccccg 120

ttcgggctcg acgtgaacgc cgcacccgca ccgatcggcc ccgacgcacc ccgcgagcag 180

cagctggagt acgcgatggg cgccgaggcc gcgttcgagg gggtgttcgc cgccctcatg 240

gacggcctcg atcccgtccc cggcatcgag cgccgcacgg agacgatcag cggcccggcc 300

ggcaacgaga tcaagctcta cgtccaccgt cccgccggag ccgtcggccc actgcccggc 360

atcttccaca tccacggcgg cggcatggtc atcctgcagg ccgccgggcc ggtctacgtg 420

cggttccgcg acgagctggc ggccaccggc acggtcgtcg tcggcgtgga gtaccgcaac 480

ggcgccggcg tgctgggccc gcacccgttc ccggccggac tccacgactg cgcggtcgcc 540

ctggactggg tgcacgcgcg acgtgcggag ctgggcatct cgacgttgac cgtcgccggc 600

gagtccggcg gcggaaacct cacgctggcg acggcgatcc gcgccaagcg cgagggcagg 660

ctcgacgcca tcgacggcgt gtacgccctc gtcccgtaca tctccggaat gtacggccgc 720

agccgcgagg agcgggaggc cgagctgccc tccctcgtgg agtgcgacgg ctacttcatc 780

tcctgcgacc tgtgcgcggt gttcgtcgag gtctacgacc cgggcacggc gcacctcacc 840

gatccgctcg cctggccgta ccacgccgcg cgggaggacc tggtcgggct gccgccgcac 900

gtgatctcgg tcaacgaggt cgacccgctg cgcgacgagg gcctggccta ctaccgcaag 960

ctcgtggagg cgggcgtcga ggcgcgctcg cgggtggtgc ccggcgcgtg ccacgccgcc 1020

gacatgatgt tccggaaggc ggcccccgac atgtacgagg cgacggtgca ggacatccac 1080

gacttcgtca cgagcctgca ccgctga 1107

<210> 11
<211> 1659
<212> DNA
<213> Pseudomonas aestusnigr

<400> 11
atgagtactg ctcgcctgac gccaaccacg gcatcaacac tcaagctgct ttccgtcgcc 60

gtcgcggccc tcacgctttc cgcctgcctg tcaggtggcg gcggtggccg tgccgacggc 120

cccaaccccc tggcgattga aaccagcgag ggtaaagtcg tcggtatcag caacgacggc 180

atccgcgtct tccgcggcat tccctacgcc gcaccgccgg ttggcgacct gcgcctcgcc 240

ccgccgcagc cacccgccag cagaagtgaa acactcagac tgagcgaaga gttcggcaac 300

agctgcccgc agtccgatct gaccactggc cagcaggtag gcaacgagga ctgcctgtac 360

ctgaacgttt atgcgcccgc tgaagcggag gatctgccgg taatggtctg gatccatggt 420

ggcgccttcg tgtttggtaa cggcggcggt gagtacgacc cgacgcgcct ggtcgaacag 480

gacgttatcg tcgtgacact caattaccgc cttggcaatc tcggttttct ggcccaccct 540

gccctggagt ccgatgccgg caactttgcc ctgatggacc agcagctcgc gctggcctgg 600

gtcaaggaaa acatcgctgc ctttggcggt gatccggcca atgtcacaat cttcggcgag 660

tccgccggtg gccatagcgt gatgagccac attgtctcgc cccgggctga agaggcagat 720

ctgttccagc gcgccattgt ccagagcggc tcctacgcgc ccttccagat gcccaaggca 780

acagcgcagt tcctcggcac atcggtagcc aatggtctcg gctgcaccga cccggagacc 840

gctgcaagct gcctgcgctc cctgccggta tccgcgttcc tggcggccca gggctcgcag 900

tcaatcccgg ttgtggatcc ggacgacgac ctgctaccca agtccatcca gcaggcactg 960

gccgacggcg acttcaacag ctcgctcgat atcatgatcg gcagcaacca gaacgagggc 1020

accctgttcg tcgcgctgga tgaagtagga ggcgatccta ttgatgacga ggcggaatac 1080

cgtgagcgcg ttgccgagtt cttccagccc taccaggcga gcatcccctt cgacgacgac 1140

cagatcgcca ctgattatct ggactttgtt gacggtgcgg caaaaccctt cgaggcagcc 1200

ctgtccggaa tctggaccga cttcatgttc gcctgcaacg cctactcgca ggccagcacc 1260

tttgccggcg ccagcatgaa taccttccag tactggttcc gcgacgagga cgccccctgg 1320

accctggtgc cgccatttgc agtgagcttc ccgctgggcg ccacccacgc tggggaaatc 1380

ccctacgtac tctacccaca ggccatcatg gagcagcgct ataccggcga tccggatgac 1440

ctgaacagtc tggccggcga gatggtcgac tactggactc aattcgccaa aaccggcgat 1500

cccaacacca ccgacggggt cgcagcagcc tggcagcagg cggcaaccgg gaacctgttg 1560

acgctggacg tacccaacgc aagcaacgca aatacgcttg gcttcctggg ctatcaccac 1620

tgcagctact gggctgaccc gccgctggta ttgccctga 1659

<210> 12
<211> 1218
<212> DNA
<213> Pseudomonas aestusnigr

<400> 12
atggcaaaca gcaactgcat cgccggcgtt gacctggaac ggctctcacg catcggcact 60

cacctgcaat cggcctatat tgacgcaggc aagctgcccg gcgctctgac tctggtggcg 120

cgccgtggcc aggttgtcta ttgcgaagca caagggctga gagacgtgga gcgcggcctg 180

ccggtcgagc gcgatacgct gttccgcatc tactcgatga ccaagccggt cacctctgtt 240

gccctgatgc agctgtacga acaaggtcgc tttctgctgg atgacccggt ccacaagtac 300

atccccagct ggagcaacct gcgcgtctac aagaccggca gccatccgca gattctgacc 360

accccctgcg agcgccccat gaccatccgt gacctgctga cccaccagtc cggtctgacc 420

tatggcttca tgaatcgcac caacgtcgac gccgcctacc gggcgctcaa actcgacggc 480

ggccccggcc tgaccctcga ccgtctgatc gacgaactgt cacggctgcc gcttgagttc 540

tcgcccggca cggcctggaa ctactcggtg gcgacagacg tctgcggtta tctggtgcaa 600

gtactgtcgg gcatgagcct ggacgactac ttcaagcgcc atatcttcga cccgctggag 660

atgagcgaca ccttcttcac cgtcccggcc gatcgcatcc cgcgcttcgc cgcctgctat 720

cagttccagc ccggcggcag cttcagtctg caggatgacc ctcaggattc cagcttcacc 780

aaggcccacg gctttctctc cggcggcggc ggtctggtct cgaccatcga cgactactac 840

cgctttgccc aggcactggc caacggtggt gaactgaacg gcgcccgcat catcggccgc 900

aagacgcttg aattcatgcg catgaaccac ctgcccggca accaggatct gcccggtgta 960

tccgtcggtt ccttcagtga aacgccctac gagggcagcg gattcggtct cggcttttca 1020

gtcaagatcg acgtcgccaa atcgcagacc aacggctccg tcggcgaata cggctggggc 1080

ggcatggcca gtaccaactt cttcgttgat ccggttgagg atctgttgat ggtgtttatg 1140

acccagctga ttccgtcgtc gagttatccg gtaaggcagg agttgcgggc gatcatcaat 1200

ggcgcgttgg tggattga 1218

<210> 13
<211> 915
<212> DNA
<213> Pseudomonas aestusnigr

<400> 13
atgccattta acaagaaaag cgttcttgcc ctctgtggcg ctggcgcgct gctgttctcg 60

atgtctgccc tggccaacaa cccggcaccg accgatccgg gtgattcggg cggtggttcc 120

gcctatcagc gcggtccgga tccgtccgtc agcttcctcg aagccgatcg cggtcagtac 180

agcgtccgtt ccagccgtgt ctccagcctg gtcagcggct ttggcggcgg caccatttac 240

tatccgacag gcacaaccgg cactatgggt gcggtcgttg taattcccgg cttcgtttct 300

gccgagtctt cgattgactg gtggggcccg aaactggcgt cctatggttt cgtggtcatg 360

accattgaca ccaacaccgg ctttgatcag ccgccgagcc gtgcgcgtca gatcaacaac 420

gcactggatt atctggttag ccagaacagc cgcagctcca gcccggttcg cggcatgatc 480

gacaccaatc gtctgggcgt cattggctgg tcgatgggcg gtggcggcac cctgcgtgtt 540

gccagtgaag gtcgtatcaa ggcagccatt ccgctggcgc cctgggatac aaccagctac 600

tacgccagcc gctcgcaggc accgactctg atctttgcct gtgagtcgga tgtgatcgcg 660

ccggtcctcc agcatgcctc accgttctac aattccctgc cttccagcat cgacaaggcc 720

tttgtggaaa tcaatggtgg cagccactac tgcggtaatg gcggcagcat ctacaacgat 780

gtgctgagcc ggttcggggt gtcctggatg aaactgcatc tggatgaaga cagccgctac 840

aagcagttcc tctgcggacc gaaccacact tccgactctc agatctccga ttatcgcggc 900

aactgcccgt actaa 915

<210> 14
<211> 657
<212> DNA
<213> Pseudomonas aestusnigr

<400> 14
atgtcaccgc acatcatcga acccgccacg cccgccaccc gcagcgtcat ctggctgcac 60

ggtctgggcg ccgactgcta cgatttcgtg ccggtagtcg aggccctgca actgcccgcc 120

gatcatgcca tccgcttcat tttccacagg gcaccgaccc ggccggtcac catcaacggc 180

gggttcccga tgccctgctg gtacgacatc ctcggcatgt cgccagcccg ggcaatcaac 240

cagagtcatc tggatgaggc cgttgccctg gtacgtcagc tgatcgacga acaatgcgcc 300

cagggcattg cgctggaaaa catcattctc gccgggttct cccagggcgg cgcggttgtg 360

ctctgcaccg ctgtcagcag cgagctgccg cttggcggtg tgatggcgct atccacctac 420

gggccgggac tggacctgct gctcgagcaa cacccggccc gccaaccgct ggagctgttc 480

tgcgcacacg gccgttttga tgacgtgctg ccattggcga tgggccgcga agcccatgac 540

ctgatgcagg ccgccggcca ccagacccgc tggtacgaat acccgatggc gcacgaggtc 600

tgcatggacg aaatcgccga catccgtcgc tggttggtcg aacgcctcgg actttga 657

<210> 15
<211> 930
<212> DNA
<213> Artificial Sequence

<220>
<223> Dim_004

<400> 15
atgtcgccgc cgcaacgcct gcgcgccggt ctcctgcgcg gctgcctgcg cctgttgttc 60

cgcgggctgg tccgtccgcc catgccagtg gcgctgcaac gcggcgtgtt gcgcgggctg 120

agcctggcga cgctgggcgc cggcggcgtc gtccgcgagc gccgcagcct cgccggcgtg 180

ccctgcgagt ggttgcggcc gcgcggggtg gaggcgcggc gccgggtgct ctacctgcac 240

ggcggcgcct acctgatcgg ctcgccggcg acccaccggg cgatcaccac gcacctggcc 300

cggcgttgcg ccgccgaggt ctgcgcggtg gactaccggc gcgccccgga acatccgttc 360

cccgccgccc gcgacgacgc cctggcggtc tacctggcat tgctggaggc cggccattcg 420

ccgcgccggc tgctgctcgc cggcgactcc gcgggcggcc acctggcgct gtccctggcc 480

ctggagctga aggcgcgcgg cttgccgttg ccggccgggt tgctgctgtt ctcgccctgg 540

accgacctcg gctgccagcg cctgcacacg ccggcggccg gcgacccgct gctgtcgcgg 600

gcctggctgg aaagcgcggt gcgcctgctg ctgccggccg acgcggagcc aggctcggcc 660

gccttgtcgc cgctgcatgc cgacctggcg gggctgccgc cgctgctggt acaggtcggc 720

gaggacgaac tgctgcgcga cgacagcctg cgcctggcgc aacgggccgg tgagcagggc 780

gtcgcggtgc gattgcagcg ctacgccggt tgctggcacg tgttccaggc tcatgccggc 840

gtgttggcca gcgccgaccg ggcgttggac gaggcggccg cgttcgtcgt cgaatgcagc 900

gcggagggcg acaggtgcag aacatcctga 930

<210> 16
<211> 1047
<212> DNA
<213> Artificial Sequence

<220>
<223> 1,4D_#003

<400> 16
atgttcgacc cggcaaggaa ccggaaatta tactttgggc atgtgttcct cgcaaggacg 60

ctcggccggg cgccgccggg cgggcccgtg ctgatcgcgc gaatcctgcg cctggccacc 120

cttgccggcg cgctgggggt gctcgggctc gccgcggtgc tggcgctggc cacgccgctc 180

ggctggccgg gcgccctcgc ctgttccatc ggggtggtgc ccgcgggctt cgcgatcgtc 240

ctcggcgtgg agttcctcgc tgccgccctg ctcggcgacc gccactggcg gggcctcgtc 300

cgcgcctggg ccggcgaggt cctggcgtcg atgcgcacct tcctcctggc cgtcccctgg 360

ctgggcgacc gggaggtcgc gagcaccgcg ggggaccggc tgcccgtcgt cctcgtgcat 420

ggctatttct gcaaccgcgc cgtgtggcga cccttcgccc gccgcctggc cgaggcgggc 480

cacccggtga cggccgtcag cctcgatccc cccttcgggc cgatcgacgc gcacgcggcc 540

acgatcgaga ccgcggtggc acgggccggc cacgggcggg ggccggtcgt gctggtcggc 600

cacagcatgg gaggcctcgc gatccgcgcc ttcctgcgcc gggcgggcag tgacggcatc 660

gcgggcgtcg tcaccctggg atccccgcac cgcggcaccc gcatggccgc ggtgggccgc 720

agcccggcgg cccgcgaaat gcgtcgcggc agcgcctggc tcgaggccct cgcggcgcac 780

gaggcacagc aacgagaggc atcgggcgat gccgctgcac ccccgctgtg catcatcctt 840

accctgaacg acaatatcgt ttatccgcag ggcgaacagg tgctgcccgg cgcgcgggtg 900

ctgcgcctgg acggcgtcgg ccacctggac ctggtctatc gcccgtccgt cgggcgcctc 960

gtggaagcgg agatcgcccg cctggaggcg ggcatcactg ccgccggcgc ccgggccgcg 1020

aacggcaagc ttgcggccgc actcgag 1047